# EUROPEAN PATENT SPECIFICATION

(11) **EP 2 361 971 B1**
(45) Date of publication and mention of the grant of the patent: **13.07.2016**
(21) Application number: 11382041.9
(22) Date of filing: 14.02.2011
(51) Int. Cl.: C12N 5/0775, A61L 27/36, A61L 27/38

(54) **Procedure for obtaining a tissue engineering product for the regeneration of bone tissue**
Verfahren zur Herstellung von Gewebezucht-Produkten zur gerichteten Regeneration von Knochengewebe
PROCÉDURE D'OBTENTION D'UN PRODUIT D'INGÉNIERIE TISSULAIRE POUR LA RÉGÉNÉRATION DU TISSU OSSEUX

(30) Priority: 19.02.2010 ES 201030238
(43) Date of publication of application: 31.08.2011
(73) Proprietor: Banc De Sang I Teixits, 08005 Barcelona (ES)
(72) Inventor: Pla Calvet, Pablo Arnau, 08173 Sant Cugat des Valles (Barcelona) (ES); Garcia Lopez, Juan, 08029 Barcelona (ES); Vives Armengol, Joaquim, 08301 Mataro (Barcelona) (ES); Caminal Bobet, Marta, 08800 Vilanova i la Geltru (Barcelona) (ES); Godia Casablancas, Francesc, 08201 Sabadell (Barcelona) (ES); Cairo Badillo, Jordi Joan, 08202 Sabadell (Barcelona) (ES); Aguirre Canyadell, Marius, 08006 Barcelona (ES)
(74) Representative: Durán Moya, Luis-Alfonso

(56) References cited:
- WO-A1-2009/080794
- WO-A2-02/070023
- WO-A2-2007/087519
- LIU GUANGPENG ET AL: "In vitro and in vivo evaluation of osteogenesis of human umbilical cord blood-derived mesenchymal stem cells on partially demineralized bone matrix.", TISSUE ENGINEERING. PART A MAR 2010 LNKD- PUBMED:19839720, vol. 16, no. 3, 18 January 2010 (2010-01-18), pages 971-982, XP002637110, ISSN: 1937-335X
- EL-SABBAN MARWAN E ET AL: "Xenogenic bone matrix extracts induce osteoblastic differentiation of human bone marrow-derived mesenchymal stem cells", REGENERATIVE MEDICINE, FUTURE MEDICINE LTD, GB, vol. 2, no. 4, 1 July 2007 (2007-07-01), pages 383-390, XP002525452, ISSN: 1746-0751, DOI: DOI:10.2217/17460751.2.4.383
- YAMADA YOICHI ET AL: "Bone regeneration following injection of mesenchymal stem cells and fibrin glue with a biodegradable scaffold.", JOURNAL OF CRANIO-MAXILLO-FACIAL SURGERY : OFFICIAL PUBLICATION OF THE EUROPEAN ASSOCIATION FOR CRANIO-MAXILLO-FACIAL SURGERY FEB 2003 LNKD- PUBMED:12553923, vol. 31, no. 1, February 2003 (2003-02), pages 27-33, XP002637111, ISSN: 1010-5182
- LIU G ET AL: "Tissue-engineered bone formation with cryopreserved human bone marrow mesenchymal stem cells", CRYOBIOLOGY, ACADEMIC PRESS INC, US, vol. 56, no. 3, 1 June 2008 (2008-06-01), pages 209-215, XP022669999, ISSN: 0011-2240, DOI: 10.1016/J.CRYOBIOL.2008.02.008 [retrieved on 2008-03-21]

## Description

This invention relates to a procedure for preparing a tissue engineering product for the regeneration of bone tissue. More particularly this invention relates to a product which mainly comprises mesenchymal cells of bone origin which have been expanded, immobilised on bony supports and combined using fibrin gels. The said procedure fundamentally comprises the stages of colonising the bony supports and combining the colonised particles through fibrin gels.

The reconstruction of bone segments because of injury, degenerative diseases, inflammation and the surgical treatment of tumours is an unresolved clinical problem. Treatments available at the present time include the transplant of autologous and heterologous bone tissue and the use of implants based on different types of biomaterials. Of the above approaches, implantation of autografts obtained from different locations, such as for example the iliac crest, is a preferred treatment for a wide variety of orthopaedic conditions. These include the replacement of bone defects and the consolidation of complicated fractures or bonding defects. The use of autologous tissue as a vehicle for bone regeneration fulfils a series of key basic requirements, provides an osteoinductive structural matrix, growth factors which promote vascularisation and osteoinduction in the implant and introduces cells with osteogenic potential.

Despite the benefits derived, from an exclusively mechanistical point of view, the use of autografts in clinical practice presents a number of major disadvantages. It frequently happens that complete replacement of the damaged bone tissue is not achieved with an autograft. These circumstances may give rise to fracture of the implant in the long term. It has been estimated that after 10 years the percentage of fractures is 60%, and these are associated with a multitude of factors such as decreased mineralisation of the newly formed bone, the production of microfractures or the activity of osteoclasts (Wheeler D.L. et al., Allograft bone decreases in strength in vivo over time. Clin. Orthop Relat Res. 2005;435:36-42). On the other hand, obtaining autologous bone surgically is frequently associated with high variable morbidity (Laurie S.W. et al., Donor-site morbidity after harvesting rib and iliac bone. Plast Reconstr Surg. 1984;6:93-98) depending upon the anatomical location and extraction technique (Chou L.B. et al., Stress fracture as a complication of autogenous bone graft harvest from the distal tibia. Foot Ankle Int. 2007;2:199-201; Brawley S.C. et al., Results of an alternative autogenous iliac crest bone graft harvest method. Orthopaedics. 2006;4:342-346.).

The use of mesenchymal stem cells on scaffolds to tissue-engineer bone has been described (LIU G ET AL, TISSUE ENGINEERING. PART A MAR 2010, vol. 16, pages 971-982; LIU G ET AL, CRYOBIOLOGY, 2008, vol. 56, pages 209-215). The use of human bone originating from tissue banks solves the problem of the morbidity associated with autografts, but lacks the benefits associated with the regenerative activity provided by the cell component, which has been determined to be a key factor. Grafts of this type are slowly incorporated into the native bone, and may end up giving rise to mechanical instability during effort or little cellular affinity, circumstances which are reflected in a lack of union with the native bone (Enneking W.F. et al., Observations on massive retrieved human allografts. J Bone Joint Surgery. 1991;73:1123-1142; Turk J.B. et al., Bone source from craniomaxillofacial reconstruction. Facial Plast Surg. 2000;16:7-14). This problem places the failure rate for this technique between 10 and 40% (Ottoleughi C.E. Massive osteo and osteo-articular bone grafts: technique and results of 62 cases. Clin Orthop Relat Res 1972;87:156-64). For this reason, although bone tissue from a bank is available, the most frequent treatment of large bony injuries is the use of an autograft.

In response to the problem mentioned this invention discloses a procedure for preparing a product based on tissue engineering, the use of which results in an effective alternative treatment. Application of the product obtained by the procedure disclosed in this invention is based on the use of matrices based on biomaterials, combined with parent cells to induce the regeneration of damaged bone tissues.

The matrices used in this invention have various properties which are key to providing the supporting function in reconstructing the target tissue. Prominent among these properties are those being biocompatible, reabsorbable, and having structural integrity and mechanical properties appropriate for the implant location. In addition to this they must be capable of giving rise to a biological environment which ensures that nutrients will be provided to the cells so that they can perform their regenerative function. In addition to the above properties it should be added that a matrix for the regeneration of bone tissues should have the ability to give rise to osteoinduction.

The second fundamental component of the tissue engineering product according to this invention is the cells. These cells also exhibit various relevant properties such as ease and safety of preparation, are a safe source from the cytogenetic point of view, and have multipotent properties which enable them to differentiate into cells responsible for the production of bone tissue, osteoblasts.

This invention therefore discloses a procedure for the preparation of a tissue engineering product intended for bone regeneration. This procedure is based on the use of expanded mesenchymal cells, of autologous or allogenic origin, obtained from bone marrow, the said cells being immobilised on supports based on deantigenised or deantigenised/demineralised bone particles. The product obtained by this procedure is combined and stabilised in the implant zone using fibrin gels. Treatment with a product obtained by the procedure according to this invention has the main advantage in comparison with treatments based on autografts that it is not necessary to subject the patient receiving the treatment to one or more major surgical operations to extract biological material.

The cellular material required for preparing the tissue engineering product according to this invention is obtained by extracting bone marrow, which can be performed on an outpatient basis. This approach therefore avoids potentially serious complications deriving from the acquisition of autologous bone material, such as chronic pain, adverse effects on nervous and arterial tissue, instability of the pelvis, infection and scarring (Banwart J.C. et al., Iliac crest bone graft harvest donor site morbidity. A statistical evaluation. Spine. 1995;20:1055-1060).

On the other hand, the procedure according to this invention, which uses mesenchymal cells and a matrix of known osteoinductive ability, preferably deantigenised or deantigenised/demineralised bone, in combination, makes it possible to overcome the limits imposed by the use of bone from a tissue bank without a cellular component, which has a bone bonding failure rate of between 10% and 40%.

The combination of demineralised/deantigenised or deantigenised bone matrices with unprocessed bone marrow has demonstrated that it accelerates and increases bone formation in comparison with the use of bone matrix alone (Rougraft B.T., Treatment of active unicameral bone cysts with percutaneous injection of demineralized bone matrix and autogenous bone marrow. J. Bone Joint Surg. Am. 2002;84:921). This result shows that bone marrow, that is to say the cells with possible osteogenic potential present in it, can be a key factor in the therapeutic activity of the product, acting as catalysts for the formation of healthy tissue.

Nevertheless bone marrow comprises a heterogeneous set of cells which include all the cells of the haematopoietic lineage, endothelial cells and adipose tissue cells, as well as a percentage of less than 0.01% of multipotent mesenchymal cells. This population of mesenchymal cells is that which gives rise to osteoblasts, the main cells responsible for bone formation, through a process of differentiation. Logically, this low percentage of mesenchymal cells in bone marrow suggests a limitation on the regenerative capacity of this source of cells. There is therefore a high degree of uncertainty over the result of treatment, as it is not possible to determine the quantity of cells having osteoinductive capacity applied and fixed in the vicinity of the injury.

The present inventors have discovered that surprising results in bone regeneration are achieved when the procedure according to this invention in which bone matrices are combined through a process of immobilising a cell product enriched in mesenchymal cells is used. This product has excellent osteoinductive capacity and is obtained through selection and subsequent *in vitro* expansion of these mesenchymal cells.

The procedure according to this invention, through manipulating the process of expanding mesenchymal cells, makes it possible to generate a wide range of amounts of bone graft, facilitating the treatment of injuries of different size and origin. This characteristic means that the product obtained through the procedure according to this invention can be applied in a range of therapeutic approaches such as the reconstruction of maxillofacial bone, and in osteoarthritis, osteonecrosis, the replacement of bone defects, intervertebral fusion, and in the consolidation of complicated fractures or bonding defects.

On the other hand, association of the mesenchymal cells with bone matrices using a process of colonisation makes it possible to locate the cells with osteogenic potential in the zone which has to be regenerated. This feature ensures reparative cell activity in the zone being treated, unlike other tissue engineering approaches which introduce the cells without immobilising them in the matrix. Furthermore, this ensures that the mesenchymal cells remain in the vicinity of the injury where they have to perform their reparative work.

Finally, combination of these particles of bone colonised with mesenchymal cells through fibrin gels makes it possible to provide the whole with plasticity and workability, in turn assisting immobilising of the mixture in the zone which has to be treated, and provision of the required architecture.

### Detailed description of the invention.

This invention discloses a procedure for the preparation of a tissue engineering product for the regeneration of bone tissue. This product comprises a cellular component consisting of expanded mesenchymal cells from bone marrow and a non-cellular component based on bone matrices. The procedure comprises the stages of:
a) expanding the mesenchymal cells,
b) associating/immobilising the mesenchymal cells obtained in (a) with bony matrices,
c) washing the bone suspension, and
d) final conditioning (combination).

The size of the injury which has to be treated will determine the quantity of mesenchymal cells required for appropriate bone regeneration. Once the required dose of mesenchymal cells has been obtained in stage (a), the stage of associating these with a bone matrix is initiated. This association-immobilisation can be carried out using dynamic or static systems.

If a dynamic system is used the mesenchymal cells obtained in expansion stage (a) are resuspended in the culture medium, preferably DMEM medium supplemented with serum in a concentration of not more than 1 x 10⁷ cells per millilitre and not less than 1 x 10³ cells per millilitre. The cellular suspension is then placed in a culture bag or flask, which may or may not be provided with a suspended stirrer which has previously been filled with bone matrix, in a sterile way. The ratio of number of cells per cubic centre of bone matrix is maintained within the range from 1 x 10² to 1 x 10⁸ cells per cubic centimetre. Once the cell suspension has been added to the flask, bioreactor or culture bag, it is incubated for 4 to 24 hours, with stirring, at a temperature of 37°C, a CO₂ saturation of 5% and a relative humidity of 95%. The stirring conditions used to ensure anchoring of the cells are 1 to 120 revolutions per minute.

If a static immobilisation system is used the mesenchymal cells obtained in expansion stage (a) are resuspended in culture medium, preferably DMEM medium supplemented with serum in a concentration of not more than 1 x 10⁷ cells per millilitre and not less than 1 x 10³ cells per millilitre. The cell suspension is then placed in a sterile way in a culture bag or flask which may or may not be provided with a suspended stirrer previously loaded with bone matrix. The ratio of number of cells per cubic centimetre of bone matrix is kept within the range from 1 x 10² to 1 x 10⁸ cells per cubic centimetre. Once the cell suspension has been added to the flask, bioreactor or culture bag, it is incubated for 4 to 24 hours under temperature conditions of 37°C, a CO₂ saturation of 5% and a relatively humidity of 95%.

The product obtained from association/immobilisation stage (b), comprising mesenchymal cells immobilised on bony matrices, preferably of deantigenised and more preferably deantigenised/demineralised bone, is subjected to a washing cycle, preferably using physiological saline solution, and is placed in a storage bag in a sterile way.

The product obtained is removed from the storage bag in a sterile way and mixed with a fibrin gel in a ratio of 1 volumetric unit of fibrinogen solution per volumetric unit of thrombin solution and volumetric unit of colonised bone matrix.

This invention will now be illustrated by means of the examples below, which do not constitute any limitation upon it.

Example 1. Procedure using dynamic immobilisation In the following procedure action was taken to obtain one cubic centimetre of tissue engineering product for bone regeneration according to this invention.

6 x 10⁶ mesenchymal cells were obtained from bone marrow by expansion using media free of animal serum and were inoculated in a culture flask. Through the use of DMEM-based medium supplemented with 10% human serum (v/v) the cell concentration was fixed at a density of 6 x 10⁵ cells per millilitre. This cell suspension was added in a sterile way to a second flask of plastics material provided with a suspended stirrer, to which 1 cubic centimetre of deantigenised bone matrix had already been added.

The flask was placed in an incubator under temperature conditions of 37°C, CO₂ saturation of 5% and a relative humidity of 95% and a stirring cycle of 24 hours duration was started at a speed of 1 rpm. Over the period of time during which this stage of immobilising the mesenchymal cells on the bone matrix took place samples of the supernatant were taken to determine the rate and yield of cell immobilisation. The immobilisation yields when this stage was complete were greater than 80% and the viability of the cells, measured by staining the nuclei or by indirect metabolic tests, was greater than 90%.

After the 24 hours had elapsed, after the mesenchymal cells had been immobilised on the bone matrix, the product obtained was washed with a view to removing cell residues and culture medium. This operation was carried out by filtration using a physiological saline solution as the washing agent. The product obtained was then placed in a sterile bag.

Before the product was applied to the patient the bone particles were combined with a fibrin gel. A volumetric ratio comprising one unit of fibrogen solution per volumetric unit of thrombin solution and volumetric unit of colonised bone matrix was used to perform this operation. This mixture was then given a shape which enabled it to fit the spatial distribution of the injury and it was positioned therein.

### Example 2. Procedure using static immobilisation

6 x 10⁶ mesenchymal cells were obtained from bone marrow by expansion using media free of animal serum and inoculated into a culture flask. Through the use of DMEM-based medium supplemented with 10% human serum (v/v) the cell concentration was fixed at a density of 6 x 10⁵ cells per millilitre. This cell suspension was added in a sterile way to a gas-permeable culture bag of plastics material to which 1 cubic centimetre of deantigenised bone matrix had previously been added.

The flask was placed in the incubator under temperature conditions of 37°C, CO₂ saturation of 5% and a relative humidity of 95% and was kept without movement for 24 hours. After the period during which this stage of immobilising the mesenchymal cells on the bone matrix had taken place samples of the supernatant were taken to determine the rate and yield of cell immobilisation. The immobilisation yields when this stage was complete were greater than 80% and the viability of the cells determined by staining the nuclei or by indirect metabolic tests was more than 90%.

After 24 hours had elapsed, after the mesenchymal cells had been immobilised on the bone matrix, the product obtained was washed to remove cell residues and culture medium. This operation was carried out by filtration using a physiological saline solution as washing agent. The product obtained was then placed in a sterile bag.

Before the product was applied to the patient the bone particles were combined with a fibrin gel. A volumetric ratio comprising one unit of fibrinogen solution per volumetric unit of thrombin solution and a volumetric unit of colonised bone matrix was used to perform this operation. The said mixture was then given a shape which enabled it to fit the spatial distribution of the injury and it was positioned therein.

## Claims

1. An in vitro procedure for the preparation of a tissue engineering product for the regeneration of bone tissues comprising the stages of :
a) selecting and expanding mesenchymal cells obtained from bone marrow,
b) associating by immobilisation of the mesenchymal cells obtained in (a) in bone matrices in a ratio in the range from 1 x 10² to 1 x 10⁸ cells per cubic centimetre in a DMEM medium supplemented with serum during a time within the range from 4 to 24 hours,
c)washing the product obtained in b), and
d)final conditioning by combination with fibrin gel.

2. A procedure according to Claim 1 in which the association/immobilisation stage is carried out by means of a dynamic system or a static system.

3. A procedure according to Claim 1 or 2, in which the bone matrix is deantigenised bone.

4. A procedure according to the preceding claims in which the bone matrix is deantigenised and demineralised bone.

5. A procedure according to the preceding claims in which the wash is performed with a physiological saline solution.

6. A procedure according to the preceding claims in which the wash is performed in cycles and the washing agent is removed by filtration.

7. A procedure according to the preceding claims in which the association/immobilisation stage (b) is carried out under the conditions of a temperature of 37°C, a CO₂ saturation of 5% and a relative humidity of 95%.

8. A procedure according to the preceding claims in which the stirring when the dynamic system is used in the association/immobilisation stage (b) is carried out within a range from 1 to 120 rpm.

9. A procedure according to the preceding claims in which the immobilisation yield from the association/immobilisation stage (b) is more than 80% and the viability of the cells determined by staining the nuclei or by indirect metabolic tests is more than 90%.

## Patentansprüche

1. In-vitro-Verfahren zur Herstellung eines Gewebezucht-Produktes zur Regeneration von Knochengewebe, wobei das Verfahren die folgenden Abschnitte umfasst:
a) Auswählen und Expandieren von aus Knochenmark gewonnenen Mesenchymzellen,
b) Assoziation durch Immobilisation der in Abschnitt a) gewonnenen Mesenchymzellen in Knochenmatrizen in einem Verhältnis im Bereich von 1 x 10² zu 1 x 10⁸ Zellen pro Kubikzentimeter in einem mit Serum supplementierten DMEM-Medium für eine Zeitdauer im Bereich von 4 bis 24 Stunden,
c) Waschen des in Abschnitt b) erhaltenen Produkts, und
d) abschließende Konditionierung durch Kombination mit Fibrin-Gel.

2. Verfahren nach Anspruch 1, bei dem der Verfahrensabschnitt zur Assoziation/Immobilisation mittels eines dynamischen oder eines statischen Systems durchgeführt wird.

3. Verfahren nach Anspruch 1 oder 2, bei dem die Knochenmatrix aus deantigenisiertem Knochen besteht.

4. Verfahren nach einem der voranstehenden Ansprüche, bei dem die Knochenmatrix aus deantigenisiertem und entmineralisiertem Knochen besteht.

5. Verfahren nach einem der voranstehenden Ansprüche, bei dem der Waschvorgang mit physiologischer Kochsalzlösung erfolgt.

6. Verfahren nach einem der voranstehenden Ansprüche, bei dem der Waschvorgang in Zyklen erfolgt und das Waschmedium durch Filtration entfernt wird.

7. Verfahren nach einem der voranstehenden Ansprüche, bei dem der Verfahrensabschnitt b) zur Assoziation/Immobilisation unter folgenden Bedingungen erfolgt: einer Temperatur von 37°C, einer CO₂-Sättigung von 5% und einer relativen Feuchtigkeit von 95%.

8. Verfahren nach einem der voranstehenden Ansprüche, bei dem der Rührvorgang bei Verwendung des dynamischen Systems im Verfahrensabschnitt b) zur Assoziation/Immobilisation in einem Drehzahlbereich von 1 bis 120 U/min erfolgt.

9. Verfahren nach einem der voranstehenden Ansprüche, bei dem das Immobilisationsergebnis aus dem Verfahrensabschnitt b) zur Assoziation/Immobilisation über 80% beträgt und die durch Färbung der Zellkerne bzw. durch indirekte metabolische Tests nachgewiesene Zellviabilität bei über 90% liegt.

## Revendications

1. Procédure in vitro pour la préparation d'un produit d'ingénierie tissulaire pour la régénération de tissus osseux, comprenant les étapes de :
(a) sélectionner et assurer l'expansion de cellules mésenchymateuses obtenues à partir de moelle osseuse,
(b) associer par immobilisation des cellules mésenchymateuses obtenues dans (a) dans des matrices osseuses selon un taux dans la plage de 1 x 10² à 1 x 10⁸ cellules par centimètre cube dans un milieu DMEM additionné d'un sérum pendant une durée comprise dans la plage de 4 à 24 heures,
(c) laver le produit obtenu dans (b), et
(d) effectuer un conditionnement final par combinaison avec un gel de fibrine.

2. Procédure selon la revendication 1 dans laquelle l'étape d'association/immobilisation est réalisée au moyen d'un système dynamique ou d'un système statique.

3. Procédure selon la revendication 1 ou 2, dans laquelle la matrice osseuse est de l'os débarrassé des antigènes.

4. Procédure selon les revendications précédentes dans laquelle la matrice osseuse est de l'os débarrassé des antigènes et déminéralisé.

5. Procédure selon les revendications précédentes dans laquelle l'étape de lavage est réalisée avec une solution saline physiologique.

6. Procédure selon les revendications précédentes dans laquelle l'étape de lavage est réalisée sous forme de cycles et l'agent de lavage est enlevé par filtration.

7. Procédure selon les revendications précédentes dans laquelle l'étape d'association/immobilisation (b) est réalisée sous les conditions d'une température de 37 °C une saturation en CO₂ de 5 % et une humidité relative de 95 %.

8. Procédure selon les revendications précédentes dans laquelle l'agitation lorsque l'on utilise un système dynamique dans l'étape d'association/immobilisation (b) est réalisée dans une plage de 1 à 120 tr/mn.

9. Procédure selon les revendications précédentes dans laquelle le rendement d'immobilisation résultant de l'étape d'association/immobilisation (b) est de plus de 80 % et la viabilité des cellules déterminée par la coloration des noyaux ou par des tests métaboliques indirects est de plus de 90 %.
